Europäisches Patentamt

European Patent Office

Office européen des brevets

(19)

(11) Veröffentlichungsnummer : **0 285 899 B1**

# (12) EUROPÄISCHE PATENTSCHRIFT

(45) Veröffentlichungstag der Patentschrift :
06.03.91 Patentblatt 91/10

(51) Int. Cl.⁵ : **C07C 69/60, C07C 67/08**

(21) Anmeldenummer : **88104542.1**

(22) Anmeldetag : **22.03.88**

(54) Verfahren zur Herstellung von Maleinsäuredimethylester.

(30) Priorität : 02.04.87 DE 3711155

(43) Veröffentlichungstag der Anmeldung :
12.10.88 Patentblatt 88/41

(45) Bekanntmachung des Hinweises auf die
Patenterteilung :
06.03.91 Patentblatt 91/10

(84) Benannte Vertragsstaaten :
AT BE CH DE ES FR GB IT LI LU NL

(56) Entgegenhaltungen :
EP-A- 0 062 874
DD-A- 229 117

(56) Entgegenhaltungen :
DE-C- 976 413
CHEMICAL ABSTRACTS, Band 106, Nr. 15, 13.
April 1987, Columbus, Ohio, USA; YOSHIDA,
HIROSHI et al.: "Purification of dialkyl maleates" Seite 589, Spalte 1, Zusammenfassung
Nr. 119301w

(73) Patentinhaber : BAYER AG
W-5090 Leverkusen 1 Bayerwerk (DE)

(72) Erfinder : Makowka, Bernd, Dr.
Leuchter Gemark 1
W-5060 Bergisch Gladbach (DE)
Erfinder : Block, Hans-Dieter, Dr.
Biesenbach 49
W-5090 Leverkusen 3 (DE)

**Beschreibung**

Die Erfindung betrifft ein kontinuierliches Herstellungsverfahren für Maleinsäuredimethylester durch Veresterung von Maleinsäureanhydrid, Maleinsäure, Maleinsäuremonomethylester oder deren Gemische mit Methanol in Gegenwart katalytischer Mengen Schwefelsäure.

Maleinsäuredimethylester ist ein vielseitig weiterverwendbares technisches Zwischenprodukt, so z.B. als Ausgangsstoff für die Copolymerisation mit anderen Vinylverbindungen und als Synthon für Pharmaka und Pflanzenschutzmittel.

Ein bekanntes Standardverfahren der Veresterung ist die Umsetzung eines Alkohols mit einer organischen Säure, meist in Gegenwart von Veresterungskatalysatoren, welche in der Regel anorganische Säuren oder Arylsulfonsäuren sind. Im Fall niedrig siedender Alkohole, wie Methanol oder Ethanol, schreitet die Veresterungsreaktion nur langsam fort, vor allem wegen der Schwierigkeit, gebildetes Reaktionswasser aus dem Gemisch zu entfernen. Aus dem gleichen Grund erhält man bei der Veresterung von Dicarbonsäuren unvollständig umgesetzte Reaktionsgemische mit meist hohen Anteilen von Monoester.

Um diese Schwierigkeiten auszuräumen, wurden häufig sehr hohe Alkoholüberschüsse eingesetzt, jedoch senkt man hierdurch Reaktionstemperaturen und Reaktionsgeschwindigkeiten in einem Maß, das wirtschaftlich nicht erwünscht ist. Der Zusatz von wasserschleppenden Hilfsstoffen, ist bei Veresterungen gebräuchlich, im Spezialfall des Methanols jedoch schlecht anwendbar, da dessen Siedepunkt zu niedrig ist.

In der deutschen Patentschrift 976 413 ist ein Verfahren zur Herstellung von Estern in kontinuierlicher Arbeitsweise beschrieben. Dabei wird das Veresterungsgemisch durch eine Reihe von Heizvorrichtungen auf einer solchen Temperatur gehalten, daß eine Fraktionierung der Reaktionsteilnehmer weitgehend vermieden wird.

Aus der deutschen Patentschrift 2 335 307 ist ein Verfahren bekannt geworden, welches die vorgenannten Hindernisse für eine große Zahl hochsiedender Carbonsäuren überwindet und bei welchem die Ester hochsiedender organischer Säuren mit leichtflüchtigen Alkoholen in Gegenwart eines Veresterungskatalysators hergestellt werden, indem man einen Überschuß des Alkohols in eine Carbonsäureschmelze einleitet und gebildetes Reaktionswasser dampfförmig aus dem Reaktionsgemisch entfernt, bis dieses im wesentlichen voll verestert und im wesentlichen frei von Wasser und Alkohol ist. Dieses einstufige und diskontinuierliche Verfahren mit Umsetzungsgraden um 90%, bezogen auf die Säure, wird bei hohen Temperaturen durchgeführt, was beispielsweise im Fall des Maleinsäuredimethylesters zu weiterreichenden Umlagerungsreaktionen in den nicht gewünschten Fumarsäuredimethylester führt.

Nach einem neueren Verfahren, welches in der deutschen Patentschrift 3 114 320 beschrieben ist, sind auch Maleinsäureester kurzkettiger Alkohole zugänglich. Dabei werden Maleinsäure, Maleinsäureanhydrid oder deren Gemische mit Überschüssen bis zu 300% eines leichtflüchtigen Alkohols unter Druck zu Maleinsäuredialkylester umgesetzt. Bei Reaktionstemperaturen, die 60-100°C über dem Siedepunkt des eingesetzten Alkohols liegen, werden zur annähernd vollständigen Veresterung Zeiten bis zu 19 Stunden benötigt. Unter diesen Bedingungen erhält man im rohen Maleinsäuredialkylester einen Fumarestergehalt bis zu 4 Gew.-%, was für viele Anwendungen von Nachteil ist.

Beispielsweise besitzt ein Gemisch aus 96 Gew.-% Maleinsäure- und 4 Gew.-% Fumarsäuredimethylester einen Schmelzpunkt von ca. 25°C, so daß dessen Handhabung im Vergleich zu reinem Maleinsäuredimethylester (Lagerung, Pumpfähigkeit) mit technischem Mehraufwand verbunden ist. Weiterhin können diese Fumaresteranteile bei Folgereaktionen zu störenden Nebenprodukten führen, was bei der großen Bedeutung des Maleinsäureesters als Zwischenprodukt besonders nachteilig ist.

Es wurde nun überraschend gefunden, daß man Maleinsäuredimethylester mit hohen Umsetzungsgraden in kurzer Zeit und mit einem Fumarsäuredimethylestergehalt unter 1 Gew.-% erhält, wenn man die Veresterungsreaktion innerhalb 20-120 Minuten bei Atmosphärendruck und relative niedrigen Temperaturen durchführt.

Des erfindungsgemäße Verfahren zur Herstellung von Maleinsäuredimethylester aus Maleinsäure, Maleinsäureanhydrid, Maleinsäuremonomethylester oder deren Gemischen mit Methanol in Gegenwart katalytischer Mengen Schwefelsäure ist dadurch gekennzeichnet, daß man die Veresterung kontinuierlich in einem Blasensäulenreaktor mit einem Methanolüberschuß von 0,4 bis 2,0 Mol Methanol pro Mol zu erzeugenden Maleinsäuredimethylester innerhalb 20-120 Minuten bei Normaldruck und Reaktionstemperaturen von 90-140°C derart durchführt, daß das Methanol vollständig oder teilweise gasförmig durch den Blasensäulenreaktor von unten nach oben und das Reaktionsgemisch aus Maleinsäuredimethylester, Maleinsäuremonomethylester, Maleinsäureanhydrid und etwa stöchiometrischer Menge Methanol sowie Katalysator von oben nach unten durch den Blasensäulenreaktor geleitet werden. Vorzugsweise wendet man Alkoholüberschüsse von 50-100%, bezogen auf die Stöchiometrie, und Verweilzeiten von 40-60 Minuten an.

Bei dem Verfahren gemäß der Erfindung werden sehr viel kürzere Reaktionszeiten und niedrigere Fumarsäureestergehalte erreicht, als nach dem Stand der Technik erwartet werden konnte.

Bei dem Verfahren der Erfindung wird das Veresterungsgemisch kontinuierlich durch mehrere Reaktionszonen des Blasensäulenreaktors geführt, bis das Gemisch frei von Wasser ist ; aus dem Reaktor wird ein Gemisch aus Maleinsäuredimethylester und Anteilen gelösten Methanols (2-4 Gew.-%) entnommen.

Unter dem Begriff Blasensäulenreaktor werden Kontaktapparate verstanden, wie sie z. B. in

1. "Reaktionstechnik in Blasensäulen", Wolf-Dieter Deckwer, Verlag Salle/Sauerländer, 1985,

2. F.J. Brück und H. Hammer, Chem. Ing. Techn. 58, Heft 1 (1986) ; Chem. Ing. Techn. MS 1432/86,

3. Ullmanns Encyklopädie der techn. Chemie, Band 3, Verfahrenstechnik II und Reaktionsapparate, Verlag Chemie, Weinheim/Bergstraße 1973, S. 369-372

4. J.R. Faif, Chem. Eng. 74, July 3, 67-74 (1967)

5. R.A. Mashelkar, Brit. Chem. Eng. 15 (10), 1297 - 1304 (1970)

6. Kirk-Othmer, Encyclopedia of chemical technology, Third Edition, John Wiley & Sons, New York-Chicheste-Brisbane-Toronto

    a) Volume 7 (1979), S. 870-875

    b) Volume 19 (1982), S. 886

beschrieben sind.

Bei dem erfindungsgemäßen Verfahren ist die Beheizung der einzelnen Reaktionszonen mittels voneinander unabhängiger Heizvorrichtungen auf eine vorzubestimmende Temperatur, bei der nämlich eine Fraktionierung der Reaktionsteilnehmer weitgehend vermieden werden soll, überhaupt nicht erforderlich ; vielmehr sind die Heizvorrichtungen in einfacher Weise miteinander verbunden und es müssen nur die selbstverständlichen Forderungen, daß die Heizquelle eine höhere Temperatur als das Reaktionsmedium haben muß, und daß die zugeführte Wärmemenge ausreichend zur Verdampfung des Reaktionswassers und des größten Teils des überschüssigen Methanols und nicht zur Verdampfung des größeren Teils des erzeugten Maleinsäuredimethylesters ausreichend sein müsse, erfüllt sein. Diese Forderungen erfüllt man in einfachster und sicherer Weise durch Heizaggregate, die mit gespanntem Dampf beschickt werden.

Erfindungsgemäß wird ein annähernd stöchiometrisches Gemisch aus Maleinsäureanhydrid, Maleinsäure und/oder Maleinsäuremonomethylester und Methanol sowie katalytische Mengen Schwefelsäure, bezogen auf die vorgenannte Reaktionsmischung, in der Regel 0,1-1 Gew.-% in die oberste Reaktionsstufe einer Blasensäule eindosiert.

Ein 50-100%iger molarer Überschuß, bezogen auf die eingesetzte Maleinsäurekomponente, von Methanoldampf, der dem ablaufenden Veresterungsgemisch entgegenströmt, wird durch Einleiten flüssigen oder dampfförmigen Methanols in der untersten Stufe erzeugt. Längs der Kolonne bildet sich ein Temperaturprofil aus, dessen Temperaturwerte zwischen 90° und 140°C liegen.

Die Einzelwerte stellen sich ein je nach der Temperatur der Heizquelle und der Wärmeaustauschfläche und der zu erhitzenden bzw. zu verdampfenden Stoffmenge. Gebildetes Reaktionswasser und geringe Mengen verdampfter Maleinsäuredimethylester werden mit überschüssigem Methanoldampf über Kopf abgetrieben. Das der untersten Reaktionszone entnommene Gemisch aus Maleinsäuredimethylester, Methanol und Katalysatorsäure wird destillativ aufgearbeitet und der Katalysator im Gemisch mit einem Teil des Esterprodukts in die Reaktion zurückgeführt.

Die Vorteile des Verfahrens sind die erreichbaren niedrigen Fumarsäureesterkonzentrationen verbunden mit hohen Umsetzungsgraden in vergleichsweise kurzen Veresterungszeiten bei geringem technischen Aufwand. Durch die Wiederverwendung der Katalysatorsäure und die Rückführung aller Wertprodukte fallen keine verschmutzten Abwässer und Abgase an, was als weiterer Verfahrensvorteil anzusehen ist.

In den folgenden Beispielen wird die Herstellung des Maleinsäuredimethylesters gemäß der Erfindung näher erläutert (%-Angaben sind soweit nicht anders angegeben Gew.-%).

## Beispiel 1

Die Veresterungsapparatur besteht aus einer vierstufigen Blasensäule aus Glas, die einen Innendurchmesser von 100 mm und eine Gesamthöhe von 1800 mm aufweist. Die Sprudelhöhe der einzelnen Reaktionszonen, die mit Dampfheizschlangen bestückt sind, beträgt 200 mm.

5,54 kg/h Maleinsäureranhydrid, 3,68 kg/h Methanol und 0,06 kg/h 98%ige Schwefelsäure werden in die oberste Stufe des Reaktors eindosiert. Das Molverhältnis Maleinsäureanhydrid zu Methanol beträgt 1 : 2,03, die Katalysatorkonzentration liegt bei 0,6 Gew.-%. In die unterste Stufe werden 2,8 kg/h extern verdampftes Methanol eingeleitet. Die insgesamt eingesetzte Alkoholmenge liegt 80% über den stöchiometrisch notwendigen Mengen. Die Reaktionstemperatur in den Reaktionszonen beträgt 100 bis 125°C, die Verweildauer des Veresterungsgemisches im Reaktor beläuft sich auf ca. 45 Minuten.

Das ablaufende, aus dem untersten Boden entnommene Esterprodukt besitzt folgende durchschnittliche Zusammensetzung :

96,5% Maleinsäuredimethylester
0,7% Schwefelsäure
0,3% Fumarsäuredimethylester
2,5% Methanol

Das über Kopf abgetriebene Dampfgemisch ist wie folgt zusammengesetzt :

55% Methanol
26% Wasser
19% Maleinsäuredimethylester.

Die in den Beispielen 2 bis 7 beschriebenen Umsetzungen wurden in der in Beispiel 1 bereits aufgeführten Apparatur durchgeführt und sind der Übersicht wegen in tabellarischer Form dargestellt.

| Beispiel-[a] Nr. | kg/h MSA | kg/h (fl.) CH$_3$OH | Überschuß[b] CH$_3$OH bzgl. MDE | Temp.- Bereich | Verweilzeit in Minuten | Umsatz bzgl. MDE | FDE-[c] Gehalt |
|---|---|---|---|---|---|---|---|
| 2 | 6,17 | 4,03 | 66 % | 92 - 98° C | 40 | 99 % | 0,1 % |
| 3 | 10,98 | 7,23 | 45 % | 90 -100° C | 25 | 95 % | 0,1 % |
| 4 | 4,12 | 2,69 | 100 % | 98 -109° C | 60 | 100 % | 0,2 % |
| 5 | 3,33 | 0 | 150 % | 93 -110° C | )60 | 100 % | 0,3 % |
| 6 | 4,12 | 2,69 | 150 % | 90 - 98° C | 60 | 98 % | 0,1 % |
| 7 | 6,17 | 4,03 | 33 % | 100 -115° C | 40 | 96 % | 0,4 % |

a) Die Schwefelsäuremenge betrug jeweils 0,5 ml H$_2$SO$_4$/mol MSA
b) Überschuß = dampfförmig eingeleitete Menge
c) im Ablauf der Blasensäule


MSA = Maleinsäureanhydrid
MDE = Maleinsäuredimethylester
FDE = Fumarsäuredimethylester

## Ansprüche

1. Verfahren zur Herstellung von Maleinsäuredimethylester durch Veresterung von Maleinsäureanhydrid, Maleinsäuremonomethylester, Maleinsäure oder deren Gemischen mit Methanol in Gegenwart katalytischer Mengen Schwefelsäure, dadurch gekennzeichnet, daß man die Veresterungsreaktion innerhalb von 20-120 Minuten in einem mehrstufigen Blasensäulenreaktor bei Normaldruck, Temperaturen von 90-140°C und Methanolüberschüssen von 0,4 bis 2,0 Mol pro Mol zu erzeugenden Maleinsäuredimethylester, durchführt.

2. Verfahren nach Anspruch 1, dadurch gekennzeichnet, daß der Methanolüberschuß 0,5 bis 1,0 Mol und die Veresterungsreaktionszeit 40-60 Minuten beträgt.

## Claims

1. A process for the production of maleic acid dimethyl ester by esterification of maleic anhydride, maleic acid monomethyl ester, maleic acid or mixtures thereof with methanol in the presence of catalytic quantities of sulfuric acid, characterized in that the esterification reaction is carried out over a period of 20 to 120 minutes in a multistage bubble-column reactor at normal pressure, at temperatures of 90 to 140°C and with methanol excesses of 0.4 to 2.0 mol per mol maleic acid dimethyl ester to be produced.

2. A process as claimed in claim 1, characterized in that the methanol excess is 0.5 to 1.0 mol and the esterification time is 40 to 60 minutes.

## Revendications

1. Procédé de préparation du maléate de diméthyle par estérification de l'anhydride maléique, du maléate de monométhyle, de l'acide maléique ou de leurs mélanges par du méthanol en présence de quantités catalytiques d'acide sulfurique, caractérisé en ce que l'on effectue la réaction d'estérification dans des durées de 20 à 120 min dans un réacteur à colonne à bulles à plusieurs étages, à pression normale, à des températures de 90 à 140°C et avec des excès de méthanol de 0,4 à 2,0 mol/mol de maléate de diméthyle à produire.

2. Procédé selon la revendication 1, caractérisé en ce que l'excès de méthanol est de 0,5 à 1,0 mol et la durée de la réaction d'estérification de 40 à 60 min.